# EUROPÄISCHE PATENTSCHRIFT

(11) **EP 0 828 523 B1**
(45) Veröffentlichungstag und Bekanntmachung des Hinweises auf die Patenterteilung: **29.08.2001**
(21) Anmeldenummer: 96909973.8
(22) Anmeldetag: 23.04.1996
(51) Int. Cl.: A61L 15/44

(54) **TRANSDERMALES THERAPEUTISCHES SYSTEM MIT DRUCKFARBENFREIER IDENTIFIKATION UND VERFAHREN ZU SEINER HERSTELLUNG**
TRANSDERMAL THERAPEUTIC SYSTEM IDENTIFIED WITHOUT PRINTING INKS AND PROCESS FOR MANUFACTURING THE SAME
SYSTEME THERAPEUTIQUE TRANSDERMIQUE A IDENTIFICATION EXEMPTE D'ENCRES D'IMPRIMERIE ET SON PROCEDE DE FABRICATION

(30) Priorität: 29.05.1995 DE 19519593
(43) Veröffentlichungstag der Anmeldung: 18.03.1998
(73) Patentinhaber: LTS Lohmann Therapie-Systeme AG, 56626 Andernach (DE)
(72) Erfinder: HORSTMANN, Michael, D-56564 Neuwied (DE); STEINBORN, Peter, D-56567 Neuwied (DE)
(74) Vertreter: Flaccus, Rolf-Dieter, Dr.
(86) Internationale Anmeldenummer: DE9600699
(87) Internationale Veröffentlichungsnummer: WO9638187

(56) Entgegenhaltungen:
- EP-A- 0 114 125
- EP-A- 0 258 521
- EP-A- 0 635 262
- WO-A-86/00536
- WO-A-90/07560
- DE-U- 9 409 784

## Beschreibung

Die Erfindung betrifft ein Transdermales Therapeutisches System zur Abgabe von Wirkstoffen über die Haut an den menschlichen Körper.
Transdermale Therapeutische Systeme (TTS) sind in der arzneilichen Therapie einer Reihe von Erkrankungen bereits im Markt eingeführt und haben sich somit in der Praxis bewährt.
Darüberhinaus sind eine Reihe verschiedener möglicher Systemaufbauten aus der Literatur bekannt (siehe hierzu zum Beispiel Y.W. Chien in A.F. Kydonieus und B. Berner (Hrsg.) "Transdermal Delivery of Drugs", S.81-100).
Unabhängig von dieser Verschiedenartigkeit der möglichen Ausführungsformen gibt es jedoch auch einige grundsätzliche Gemeinsamkeiten zwischen den zur Zeit bekannten Systemen:
1. Zum Schutz vor der unerwünschten Abgabe von Wirkstoff oder auch Hautfeuchtigkeit durch das Transdermale Therapeutische System nach außen, des weiteren auch zum Schutz vor dem Ankleben an Textilien wird eine im wesentlichen undurchlässige, nicht klebende Rückschicht (1) verwendet.
2. Da Transdermale Therapeutische Systeme auf der Haut haften sollen, wird die der Haut zugewandte Schicht, zuweilen auch nur ein Teil der Kontur selbstklebend ausgerüstet.
3. Wegen dieser selbstklebenden Eigenschaften ist zur Lagerung vor Gebrauch eine wiederablösbare Schutzschicht (3), gegebenenfalls dehäsiv ausgerüstet aufgebracht.

Die Rückschicht besteht in der Regel aus pharmazeutisch üblichen Materialien wie Kunststoffolien, aber auch Papiere, Vliesstoffe oder Textilien kommen in Frage. Häufig werden wegen ihrer leichten Verarbeitbarkeit thermoplastische Kunststoffe verwendet, sei es als z.B. extrudierte oder gegossene, evtl. längs oder quer gereckte Folien, oder auch in der Form von Fasern beim Einsatz in vliesartigen oder textilen Anwendungen.

Als besonders geeignete Kunststoffe seien hier Polyethylenterephthalat (PETP) und andere Polyester, Polyethylen (HDPE oder LDPE), Polyvinylchlorid (PVC, optional weichgemacht), Ethylen-Vinylacetat-Copolymere (EVA), Polypropylen (PP) beispielhaft genannt. Zur Kombination von vorteilhaften Merkmalen sind auch Laminate im Einsatz (zum Beispiel solche aus EVA bzw. PE außenseitig und PETP). Auf diese Weise kann die Oberfläche (4) (PE oder EVA) mit hautähnlichweichem "Griff" ausgestattet werden (EVA bzw. PE) und andererseits zum aktiven Teil des Transdermalen Therapeutischen Systems hin eine PETP-Schicht (5) als Diffusionsbarriere wirken.

Ähnlich wie bei herkömmlichen Arzneiformen ergibt sich von Seiten der vertreibenden Firmen, der Arzneimittelzulassungsbehörden oder ganz allgemein von Seiten der Verbraucherverbände der Wunsch oder sogar die obligatorische Verpflichtung, eine eindeutige Kennzeichnung der Systeme vorzunehmen. Auf diese Weise soll eine sichere Identifizierung des Produktes auch dann möglich sein, wenn Packungsbeilagen und sonstige Begleitinformationen nicht vorliegen. Form, Größe und Aussehen allein können dies allein nicht sicherstellen.

Es ist daher mittlerweile üblich geworden, solche Systeme auf der Außenseite der Rückschicht mit einer geeigneten Druckfarbe zu bedrucken. Diese Möglichkeit, beschrieben in EP 0 114 125, erlaubt zwar die Identifizierung und macht farblich auffallende Gestaltungsweisen möglich.

Aus der Kunststofftechnologie ist fernerhin bekannt, daß thermoplastische Kunststoffe in der Hitze verformbar sind und diese Formänderung zur Einprägung von Mustern oder Informationen auf Gebrauchsartikeln, auch Kunststoffolienlaminaten nutzbar ist (siehe z.B. US 4 359 442).
Zur Kennzeichnung von Artikeln, insbesonderer auch der Verpackung von pharmazeutischen Artikeln, ist jedoch allenfalls Transferdruck üblich, bei welchem unter Hitzeeinwirkung mit einer beheizten Form ein pigmentiertes Folienlaminat kurzzeitig gegen den zu bedruckenden Gegenstand gepreßt wird. Auf diese Weise wird Farbpigment auf das Substrat gebracht.

Auch das "Prägen" ist grundsätzlich als Verfahren zur äußerlichen Kennzeichnung transdermaler therapeutischer Systeme bekannt (DE-Gbm 94 09 784). Hier werden folienförmige Kennzeichnungsmittel mit darauf ausgeführter Prägung oder Bedruckung auf den wirkstoffführenden Teil von wirkstoffhaltigen Pflastern angebracht.

Dieser Stand der Technik ist dennoch mit einer Reihe von Nachteilen behaftet:
- Bei der Benutzung von (nur toxikologisch einwandfreien) Druckfarben ist der Umgang mit lösungsmittelbehafteten Trägerflüssigkeiten für Pigmente und Lacke am Ort der Herstelung von Pharmaprodukten wegen der möglichen Kontamination mit Fremdstoffen nicht unproblematisch.
   Eine Reihe von Lacken haftet nicht oder nur unzureichend auf dem Rückschichtmaterial; sodaß nur bedruckungsgeeignete Materialien in Frage kommen.
   Unter dem Einfluß von flüchtigen Wirkstoffen kann die Druckfarbe auf den Transdermalen Therapeutischen Systemen während der Lagerung nachträglich erweichen und dadurch der Aufdruck unleserlich werden.
   Fernerhin ist die farblich hervortretende Codierung auf dem Transdermalen Therapeutischen System ist für den Konsumenten kosmetisch störend.
- Wird, wie in DE-Gbm 94 09 784 Prägung zur Anbringung der Codierung gewählt, muß bei Anwendung gewöhnlicher, dem Fachmann zugänglicher Techniken der arzneistofführende Bereich einem unzulässig hohen, die Arzneiform schädigenden Druck erzeugt werden. Kompression der Matrix führt auf diese Weise leicht zu lokalen Veränderungen der Funktion des Pflasters, insbesondere der Freisetzung in vitro. Eine vor Anbringung eines folienförmigen Kennzeichnungsträgers erzeugte Prägung erfordert eine weitere selbstklebende Zwischenschicht zur erleichterten Anbringung und führt fast zwangsläufig infolge Folienverformung zu einem blasigunansehnlichen Erscheinungsbild.
   Die in in DE-Gbm 94 09 784 genannte "Prägung" wird technisch im Detail zwar dort nicht näher beschrieben. Bei Verwendung einer (weit überwiegend verwendeten) Rückschicht aus einem einheitlichen Polymermaterial ist eine wie auch immer ausgeführte Prägung mit oder ohne Hitzeeinwirkung mit der Bildung "dünner Stellen" verbunden. Dies kann die Barriereeigenschaft der Rückschicht für den Wirkstoff zunichte machen und wird offenbar aus diesem Grund in der Praxis nicht eingesetzt.

Aufgabe dieser Erfindung ist daher ein Transdermales Therapeutisches System bestehend aus einer Rückschicht aus thermoplastischem Material bestehend aus einem Laminat aus einem außenseitig liegenden niedrigschmelzenden Thermoplasten und einem zu Haut hin gewandten höher schmelzenden Thermoplasten, und einer auf dieser angebrachten dauerhaften identifizierenden Codierung, sowie einem aktiven, den Wirkstoff enthaltenden Teil, welches ohne Verwendung weiterer Zusatzstoffe eine sichere Kennzeichnung auf besagter Rückschicht aufweist.

Diese Aufgabe wird erfindungsgemäß dadurch gelöst, daß diese Codierung in einer lokal unterschiedlichen Oberflächenbeschaffenheit, Oberflächendicke oder Oberflächenrauhigkeit der Rückschicht besteht.

Die Vorteilhaftigkeit des Erfindungsgegenstandes kann insbesondere bei Wirkstoffen oder auch anderen Zusatzstoffen beobachtet werden, die bei der vorgesehenen Lagertemperatur flüchtig sind und daher über den Dampfraum in die dem Stand der Technik entsprechenden Lackbestandteile einwandern könnten.
Solche Stoffe sind zum Beispiel Nicotin, Nitroglycerin, als Beispiele für pharmazeutische Wirkstoffe. Ethanol, Propandiol und andere niedermolekulare Alkohole, Menthol, Eucalyptol, Limonen und viele andere Terpene, niedermolekulare Fettsäuren wie z.B. Caprinsäure, Dimethylsulfoxid seien exemplarisch für typische Zusatzstoffe in transdermalen Therapeutischen Systemen genannt, die für die klassische Druckfarbentechnik einen Risikofaktor darstellen.
Überraschenderweise tritt nicht, wie vom Fachmann zu erwarten wäre, eine nachteilige Beeinflussung des Wirkstoffgehaltes durch die Temperatureinwirkung ein. Insbesondere bei Verwendung von modernen Druckwerken lassen sich bei kurzen Kontaktzeiten Beeinflussungen des Inhaltsstoffgehaltes praktisch ausschließen.

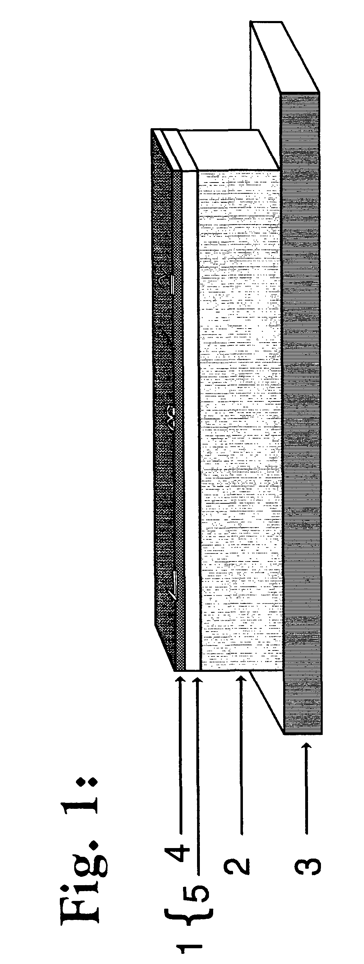

Die Erfindung ist in Fig. 1 im einzelnen dargestellt. Anstelle der abgebildeten Diffusionsmatrix (2) können ohne Beeinträchtigung der erfinderischen Lösung auch andere für solche Arzneiformen typische Elemente und Elementgruppen (Reservoire, Diffusionsmembranen, etc.) treten.
Fig. 1 zeigt im Querschnitt ein Transdermales Therapeutisches System mit in bedruckbare thermoplastische Schicht (4) und Diffusions-(und Temperatur)barriere (5) aufgeteilter Rückschicht (1).
Es enthält mithin die Elemente:
1 Rückschicht, bestehend aus (4) und (5), mit Codierung
2 Matrix
3 wiederablösbare Schutzfolie
4 thermoplastischer Teil der Rückschicht mit Aufdruck
5 Diffusionsbarriere für den Wirkstoff (PETP)

## Patentansprüche

1. Transdermales Therapeutisches System mit Rückschicht aus thermoplastischem Material,
bestehend aus einem Laminat aus einem außenseitig liegenden niedrigschmelzenden Thermoplasten und einem zu Haut hin gewandten höher schmelzenden Thermoplasten und einer auf dieser Rückschicht angebrachten dauerhaften identifizierenden Codierung,
sowie einem aktiven, den Wirkstoff enthaltenden Teil,
**dadurch gekennzeichnet**, daß diese Codierung der Rückschicht in einer lokal unterschiedlichen Oberflächenbeschaffenheit, Oberflächendicke oder Oberflächenrauhigkeit dieser Rückschicht besteht.

2. Transdermales Therapeutisches System nach Anspruch 1,
**dadurch gekennzeichnet**, daß diese Codierung durch Einwirkung von Druck, Hitze, Ultraschall oder Abrasion erzeugt wird.

3. Herstellungsverfahren für Transdermale Therapeutische Systeme nach Anspruch 1 oder 2, **dadurch gekennzeichnet**, daß die Codierung nach Fertigstellung der übrigen Herstellungsschritte am fertigen System erfolgt.

## Claims

1. Transdermal therapeutic system, with backing layer of thermoplastic material,
consisting of a laminate of a low-melting thermoplastic material on the external surface and a higher melting thermoplastic material facing the skin side, and a persistent, identifying code applied on this laminate, as well as an active part containing the active agent,
**characterized in that** this coding consists of a locally different surface property, surface thickness or surface roughness of this backing layer.

2. Transdermal therapeutic system according to claim 1, **characterized in that** this coding is generated by influence of pressure, heat, ultrasound or abrasion.

3. Method for manufacture for transdermal therapeutic systems according to claim 1 or 2, **characterized in that** the coding is made on the ready system after finishing the other steps of manufacture.

## Revendications

1. Système thérapeutique transdermique avec couche dorsale en matériau thermoplastique,
constitué d'un stratifié d'un thermoplastique à bas point de fusion situé du côté extérieur et d'un thermoplastique à plus haut point de fusion tourné vers la peau, et d'un codage d'identification appliqué de manière durable sur cette couche dorsale,
ainsi que d'une partie active contenant le produit actif,
**caractérisé en ce que** ce codage de la couche dorsale est constitué d'une modification locale de la texture, de l'épaisseur ou de la rugosité de la surface de cette couche dorsale.

2. Système thérapeutique transdermique selon la revendication 1, **caractérisé en ce que** ce codage est créé par action de la pression, de la chaleur, d'ultrasons ou d'une abrasion.

3. Procédé de fabrication de systèmes thérapeutiques transdermiques selon la revendication 1 ou 2, **caractérisé en ce que** le codage s'effectue sur le système terminé, après la réalisation des autres étapes de fabrication.
